Europäisches Patentamt

European Patent Office

Office européen des brevets

(19)

(11) Veröffentlichungsnummer : **0 156 098**
**B1**

(12) **EUROPÄISCHE PATENTSCHRIFT**

(45) Veröffentlichungstag der Patentschrift :
02.11.89

(51) Int. Cl.⁴ : **A 61 M 35/00**, A 61 M   5/18

(21) Anmeldenummer : 84890253.2

(22) Anmeldetag : 21.12.84

(54) Vorrichtung zur Applikation eines Gewebeklebstoffes.

Teilanmeldung 88121701.2 eingereicht am 21/12/84.

(30) Priorität : 29.03.84 AT 1063/84

(43) Veröffentlichungstag der Anmeldung :
02.10.85 Patentblatt 85/40

(45) Bekanntmachung des Hinweises auf die Patenterteilung : 02.11.89 Patentblatt 89/44

(84) Benannte Vertragsstaaten :
AT BE CH DE FR GB IT LI LU NL SE

(56) Entgegenhaltungen :
AT--B--   366 916
DE--U-- 8 119 976
US--A-- 2 112 160
US--A-- 4 406 656
GASTROENTEROLOGY, Band 77, Nr. 4, Teil 1, Oktober 1979, Seiten 642-646; W.G. LINSCHEER et al.:
"Control of upper gastrointestinal hemorrhage by
endoscopic spraying of clotting factors"

(73) Patentinhaber : IMMUNO Aktiengesellschaft für che-
misch-medizinische Produkte
Industriestrasse 72
A-1220 Wien (AT)

(72) Erfinder : Redl, Heinz, Dr.
Windmühlgasse 7
A-1060 Wien (AT)
Erfinder : Habison, Georg, Dr.
Taubstummengasse 15/12
A-1040 Wien (AT)

(74) Vertreter : Wolfram, Gustav, Dipl.-Ing.
Schwindgasse 7 P.O. Box 205
A-1041 Wien (AT)

EP 0 156 098 B1

## Beschreibung

Die Erfindung betrifft eine Vorrichtung zur Applikation eines Gewebeklebstoffes auf Basis von menschlichen oder tierischen Proteinen zum nahtlosen bzw. nahtunterstützenden Verbinden von menschlichen oder tierischen Gewebe- oder Organteilen, zur Wundversiegelung, Blutstillung u. dgl., welcher Gewebeklebstoff durch Zusammenbringen von Lösungen der Proteine und blutgerinnungsfördernder Gerinnungsfaktoren in situ gebildet wird, mit einer Mehrzahl von in Anschlußstücken endigenden Behältern für die Aufnahme der zu applizierenden Komponenten, vorteilhaft mit einer Mehrzahl von genormten, in Konussen endigenden Einwegspritzenkörpern aus Kunststoffmaterial, mit einem Verbindungskopf, der auf die Anschlußstücke der Behälter aufsetzbar ist und der für jede der zu applizierenden Komponenten einen eigenen zur Stirnseite des Verbindungskopfes führenden Förderkanal aufweist, und mit einer auf den Verbindungskopf aufsetzbaren Mischkanüle.

Eine bekannte Vorrichtung dieser Art ist in der AT-B-366.916 beschrieben. Als Komponenten des Gewebeklebstoffes können einerseits eine Faktor XIII und Fibrinogen enthaltende Lösung und andererseits eine Thrombin enthaltende Lösung verwendet werden. Diese Komponenten werden in einer auf den Verbindungskopf aufgesetzten Mischkanüle vermischt und auf die zu behandelnde bzw. zu schützende Wundstelle aufgebracht.

Obwohl sich die bekannte Vorrichtung im wesentlichen bewährt hat, kann sich als Schwierigkeit ergeben, daß es bei einer Unterbrechung des Flusses der Komponenten während der Applikation oder bei Verwendung langer und dünner Mischkanülen zu einer Verfestigung des Gewebeklebstoffes schon in der Mischkanüle kommt, was zur Folge hat, daß die Mischkanüle sofort ausgewechselt werden muß. Ein derartiger Vorgang kann zu erheblichen Störungen während eines Operationsablaufes führen.

Eine modifizierte Ausführungsform der in der AT-B-366.916 beschriebenen Einrichtung umfaßt eine Sprüheinrichtung für die zu mischenden Komponenten, wobei ein Zuführungskanal für ein steriles Gas im Winkel zu den Förderkanälen der zu mischenden Komponenten angeordnet ist. Bei dieser Sprüheinrichtung erfolgt eine kegelförmige Zerstäubung der Komponenten in einem Abstand von 10 bis 20 cm vor der Mündung der Förderkanäle. Diese Einrichtung ist daher nur für großflächige Auftragungen geeignet.

Die Erfindung bezweckt die Vermeidung der geschilderten Nachteile und Schwierigkeiten und stellt sich die Aufgabe, eine Vorrichtung der eingangs bezeichneten Art störungssicher zu gestalten, wobei eine während einer Behandlung notwendige Unterbrechung des Flusses der zu mischenden Komponenten keine Auswechslung von Teilen der Vorrichtung erforderlich macht.

Diese Aufgaben der Erfindung werden bei einer Vorrichtung der eingangs bezeichneten Art dadurch gelöst, daß der Verbindungskopf einen zusätzlichen Förderkanal für ein medizinisches Gas besitzt, der eng benachbart zu den übrigen Förderkanälen zur Stirnseite des Verbindungskopfes führt.

Nach einer vorteilhaften Ausführungsform weist die Mischkanüle eine mit Erhöhungen bzw. Vertiefungen versehene, die Turbulenz der durchströmenden Komponenten fördernde Innenfläche auf.

Bei der erfindungsgemäßen Vorrichtung kann es zu keiner Verstopfung kommen. Bei Erhöhung des Druckes des medizinischen Gases kann eine Zerstäubung der Komponenten an der Spitze der Mischkanüle bewerkstelligt werden, so daß die Vorrichtung auch für kleinflächige Applikationsbereiche eingesetzt werden kann.

Die erfindungsgemäße Vorrichtung ist an einem Ausführungsbeispiel in der Zeichnung näher erläutert, wobei Fig. 1 eine teilweise geschnittene Seitenansicht der Vorrichtung mit aufsetzbarer Mischkanüle, Fig. 2 einen Teilschnitt der Vorrichtung und Fig. 3 schaubildlich eine besondere Ausführungsform einer Mischkanüle zeigen.

Mit 1 und 2 sind zwei genormte Einwegspritzenkörper bezeichnet, von denen einer zur Aufnahme einer Thrombin enthaltenden Lösung und der zweite zur Aufnahme einer Faktor XIII und Fibrinogen enthaltenden Lösung dient. Die Spritzenkörper 1, 2 sind zweckmäßig als genormte Einwegspritzenkörper aus Kunststoff ausgebildet. Sie sind gemeinsam in eine Halteeinrichtung 3 eingesetzt. Diese weist zwei U-förmig gestaltete Rinnen 4, 5 auf, die jeweils an ihren Enden mit Noppen 6 ausgestattet sind, so daß die Spritzenkörper 1, 2, die von oben in die Rinnen eingesetzt werden, einrasten und durch die Noppen festgehalten werden.

Am Ende der Halteeinrichtung sind Fingergriffe 8 vorgesehen, die U-förmig gestaltete Erweiterungen 9, 10 aufweisen, in die die Flanschenden 11, 12 der Spritzenkörper ragen, so daß die Spritzenkörper in Richtung ihrer jeweiligen Längsachse 13 fixiert sind. Zwischen den U-förmigen Rinnen 4, 5 ist eine Ausnehmung 14 für eine Führungsstange 15 vorgesehen, die im Bereich der Fingergriffe 8 die Bohrung 16 der Halteeinrichtung durchsetzt. Die Führungsstange kann mit einer gemeinsamen Betätigungseinrichtung 17 für die Daumenaufsätze 18, 19 der Kolben 20, 21 verbunden sein. Diese Kolben können aber auch jeweils für sich betätigt werden.

Die beiden Konusse 25, 26 der Spritzenkörper ragen in entsprechend geformte Ausnehmungen des Verbindungskopfes 27 und sind mit diesem verbunden. Innerhalb des Verbindungskopfes führt von jedem Einsteckkonus 25, 26 ein eigener Förderkanal 28, 29 an die Stirnseite des Verbindungskopfes. Außerdem ist im Verbindungskopf ein weiterer Förderkanal 30 für ein medizinisches Gas vorgesehen, der ebenfalls an die Stirnseite des Verbindungskopfes führt, parallel und eng benachbart mit den Förderkanälen 28 und 29.

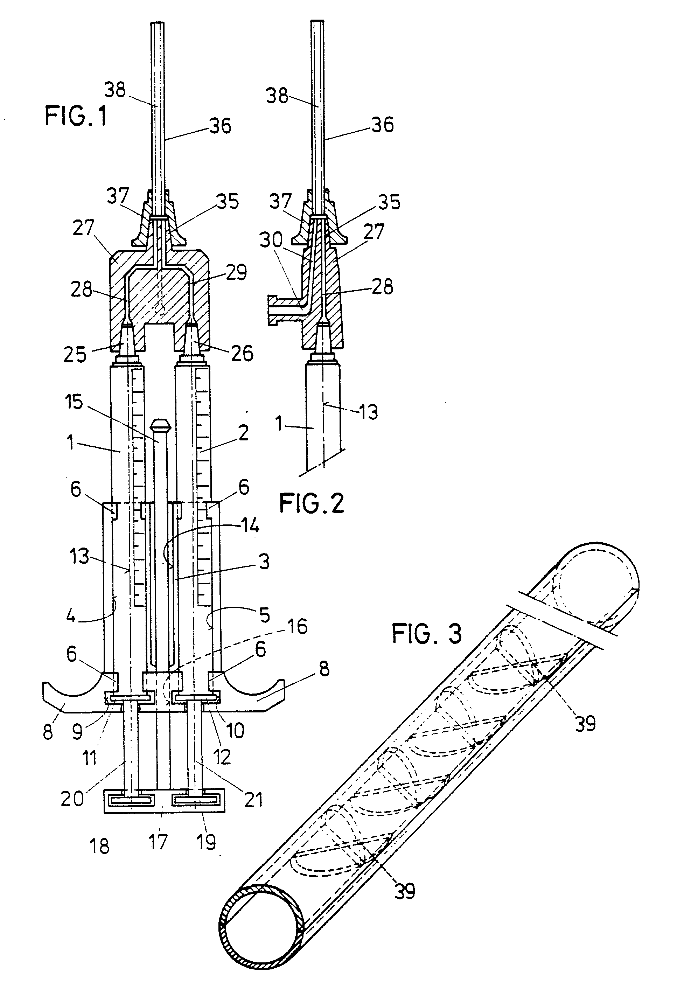

Der Verbindungskopf 27 weist einen sockelförmigen Fortsatz 35 auf, in dem die drei parallel laufenden Förderkanäle 28, 29 und 30 vereinigt sind. Auf diesen Fortsatz 35 ist eine Mischkanüle 36 mittels eines den Sockel 35 umfassenden Ansatzstückes 37 aufsetzbar. Der Innenraum 38 der Mischkanüle kann mit einer die Turbulenz der durchströmenden Komponenten fördernden Innenfläche versehen sein, wie dies in vergrößertem Maßstab in Fig. 3 gezeigt ist, wobei die die Turbulenz fördernden Einrichtungen als Nuten 39 ausgestaltet sind.

Durch Druck auf die Kolben 18, 19 bzw. die Betätigungsvorrichtung 17 nach vorherigem Öffnen eines (die Förderleitung 30 betätigenden) nicht dargestellten Ventils werden die zu vermischenden Komponenten der Applikationsstelle zugeführt, wobei stetig strömendes Gas die Förderkanäle zuverlässig freihält.

Je nach Wahl der Fördergeschwindigkeit und Fördermenge des medizinischen Gases bei der Applikation kann an der Mündung ein flüssiger Austritt der Komponenten bzw. eine Zerstäubung der Komponenten erreicht werden, so daß die Vorrichtung in weitem Umfang anwendbar ist.

**Patentansprüche**

1. Vorrichtung zur Applikation eines Gewebeklebstoffes auf Basis von menschlichen oder tierischen Proteinen zum nahtlosen bzw. nahtunterstützenden Verbinden von menschlichen oder tierischen Gewebe- oder Organteilen, zur Wundversiegelung, Blutstillung u. dgl., welcher Gewebeklebstoff durch Zusammenbringen von Lösungen der Proteine und blutgerinnungsfördernder Gerinnungsfaktoren in situ gebildet wird, mit einer Mehrzahl von in Anschlußstücken (25, 26) endigenden Behältern (1, 2) für die Aufnahme der zu applizierenden Komponenten, vorteilhaft mit einer Mehrzahl von genormten, in Konussen endigenden Einwegspritzenkörpern aus Kunststoffmaterial, mit einem Verbindungskopf (27), der auf die Anschlußstücke (25, 26) der Behälter (1, 2) aufsetzbar ist und der für jede der zu applizierenden Komponenten einen eigenen zur Stirnseite des Verbindungskopfes führenden Förderkanal (28, 29) aufweist, und mit einer auf den Verbindungskopf aufsetzbaren Mischkanüle, dadurch gekennzeichnet, daß der Verbindungskopf (27) einen zusätzlichen Förderkanal (30) für ein medizinisches Gas besitzt, der eng benachbart zu den übrigen Förderkanälen zur Stirnseite des Verbindungskopfes (27) führt.

2. Vorrichtung nach Anspruch 1, dadurch gekennzeichnet, daß die Mischkanüle (36) eine mit Erhöhungen bzw. Vertiefungen (39) versehene, die Turbulenz der durchströmenden Komponenten fördernde Innenfläche aufweist.

**Claims**

1. Arrangement for applying a tissue adhesive based on human or animal proteins for seamlessly or seam-supportingly connecting human or animal tissue or organ parts, for sealing wounds, stopping bleedings and the like, which tissue adhesive is formed in situ by bringing together solutions of the proteins and blood clot-promoting coagulation factors, with a plurality of containers (1, 2) ending in joining pieces (25, 26) and adapted to accommodate the components to be applied, advantageously with a plurality of standardised disposable syringe bodies made of synthetic material and ending in coni, with a connecting head (27) attachable to the joining pieces (25, 26) of the containers (1, 2) and having a separate conveying channel (28, 29), leading to the front side of the connecting head, for each of the components to be applied, and with a mixing cannula attachable to the connecting head, characterised in that the connecting head (27) possesses an additional conveying channel (30) for a medicinal gas, which, closely adjacent to the other conveying channels, leads to the front side of the connecting head (27).

2. Arrangement according to claim 1, characterised in that the mixing cannula (36) comprises an internal surface including elevations and depressions (39), respectively, promoting the turbulence of the components flowing therethrough.

**Revendications**

1. Dispositif pour l'application d'une colle pour tissu à base de protéines humaines ou animales pour la liaison, sans couture ou en soutien d'une couture, de parties de tissu ou d'organe humain ou animal, pour la fermeture d'une blessure, pour l'hémostase du sang et analogues, colle pour tissu qui se forme in situ par apport commun de solutions des protéines et de facteurs favorisant la coagulation du sang, comportant une pluralité de récipients (1, 2), se terminant en raccords (25, 26), pour recevoir les composants à appliquer, comportant avantageusement une pluralité d'organes d'injection jetables, en plastique, normalisés, se terminant en cône, comportant une tête de liaison (27) qui peut se placer sur les raccords (25, 26) des récipients (1, 2) et qui présente, pour chacun des composants à appliquer, son propre canal de transport (28, 29) qui conduit au côté frontal de la tête de liaison, et comportant une canule de mélange que l'on peut rapporter sur la tête de liaison, caractérisé en ce que la tête de liaison (27) possède un canal de transport supplémentaire (30) pour un gaz médicinal, qui conduit très près des autres canaux de transport en direction du côté frontal de la tête de liaison (27).

2. Dispositif selon la revendication 1, caractérisé en ce que la canule de mélange (36) présente une surface intérieure munie de renflements ou de creux (39) favorisant la turbulence des composants qui la parcourent.

FIG.1

38
36
37
27
28
25
15
1
2
6
6
13
14
4
3
5
6
6
16
8
8
9
10
11
12
20
21
18
17
19
29
26

38
36
37
35
30
27
28
1
13

FIG.2

FIG. 3

39
39